# EUROPÄISCHE PATENTANMELDUNG

(11) **EP 3 698 779 A1**
(43) Veröffentlichungstag der Anmeldung: **26.08.2020**
(21) Anmeldenummer: 19214768.4
(22) Anmeldetag: 26.08.2009
(51) Int. Cl.: A61K 31/135, A61K 31/155, A61K 31/357, A61K 31/381, A61K 31/4164, A61K 31/443, A61K 31/445, A61K 31/4743, A61K 31/496, A61K 31/506, A61P 13/02, A61P 35/00, A61P 29/00, A61G 9/00, A61K 6/00, A61K 8/00, A61Q 11/02, A61Q 17/02, A23G 9/00

(54) **NACHWEIS UND VERWENDUNG NIEDERMOLEKULARER MODULATOREN DES KÄLTE-MENTHOL-REZEPTORS TRPM8**

(30) Priorität: 26.08.2008 EP 08162997
(62) Teilanmeldung aus: 16175305.8
(71) Anmelder: BASF SE, 67056 Ludwigshafen am Rhein (DE)
(72) Erfinder: SUBKOWSKI, Thomas, 68526 Ladenburg (DE); BOSCHWEILER, Claus, 69118 Heidelberg (DE); WITTENBERG, Jens, 67117 Limburgerhof (DE); KROHN, Michael, 64653 Lorsch (DE); ZINKE, Holger, 64673 Zwingenberg (DE)
(74) Vertreter: Reitstötter Kinzebach

(57) **Zusammenfassung**

Die Erfindung betrifft neuartige Modulatoren des Kälte-Menthol-Rezeptors TRPM8, Verfahren zur Modulation des TRPM8-Rezeptors unter Verwendung dieser Modulatoren; die Verwendung der Modulatoren zur Induktion von Kältegefühl; sowie die unter Verwendung dieser Modulatoren hergestellten Gegenstände und Mittel.

SEQUENCE LISTING

<110>  BASF SE

<120>  Nachweis und Verwendung niedermolekularer Modulatoren des
       Kälte-Menthol-Rezeptors TRPM8

<130>  M/49197-PCT

<160>  2

<170>  PatentIn version 3.3

<210>  1
<211>  5621
<212>  DNA
<213>  Homo sapiens

<400>  1
aagaaaatcc tgcttgacaa aaaccgtcac ttaggaaaag atgtcctttc gggcagccag      60

gctcagcatg aggaacagaa ggaatgacac tctggacagc acccggaccc tgtactccag     120

cgcgtctcgg agcacagact tgtcttacag tgaaagcgac ttggtgaatt ttattcaagc     180

aaattttaag aaacgagaat gtgtcttctt taccaaagat tccaaggcca cggagaatgt     240

gtgcaagtgt ggctatgccc agagccagca catggaaggc acccagatca accaaagtga     300

gaaatggaac tacaagaaac acaccaagga atttcctacc gacgcctttg gggatattca     360

gtttgagaca ctggggaaga aagggaagta tatacgtctg tcctgcgaca cggacgcgga     420

aatcctttac gagctgctga cccagcactg gcacctgaaa acacccaacc tggtcatttc     480

tgtgaccggg ggcgccaaga acttcgccct gaagccgcgc atgcgcaaga tcttcagccg     540

gctcatctac atcgcgcagt ccaaaggtgc ttggattctc acgggaggca cccattatgg     600

cctgatgaag tacatcgggg aggtggtgag agataacacc atcagcagga gttcagagga     660

gaatattgtg gccattggca tagcagcttg gggcatggtc tccaaccggg acaccctcat     720

caggaattgc gatgctgagg gctatttttt agcccagtac cttatggatg acttcacaag     780

agatccactg tatatcctgg acaacaacca cacacatttg ctgctcgtgg acaatggctg     840

tcatggacat cccactgtcg aagcaaagct ccggaatcag ctagagaagt atatctctga     900

gcgcactatt caagattcca actatggtgg caagatcccc attgtgtgtt ttgcccaagg     960

aggtggaaaa gagactttga aagccatcaa tacctccatc aaaaataaaa ttccttgtgt    1020

ggtggtggaa ggctcgggcc agatcgctga tgtgatcgct agcctggtgg aggtggagga    1080

tgccctgaca tcttctgccg tcaaggagaa gctggtgcgc tttttacccc gcacggtgtc    1140

ccggctgcct gaggaggaga ctgagagttg gatcaaatgg ctcaaagaaa ttctcgaatg    1200

ttctcaccta ttaacagtta ttaaaatgga agaagctggg gatgaaattg tgagcaatgc    1260

catctcctac gctctataca aagccttcag caccagtgag caagacaagg ataactggaa    1320

tgggcagctg aagcttctgc tggagtggaa ccagctggac ttagccaatg atgagatttt 1380

caccaatgac cgccgatggg agtctgctga ccttcaagaa gtcatgttta cggctctcat 1440

aaaggacaga cccaagtttg tccgcctctt tctggagaat ggcttgaacc tacggaagtt 1500

tctcacccat gatgtcctca ctgaactctt ctccaaccac ttcagcacgc ttgtgtaccg 1560

gaatctgcag atcgccaaga attcctataa tgatgccctc ctcacgtttg tctggaaact 1620

ggttgcgaac ttccgaagag gcttccggaa ggaagacaga aatggccggg acgagatgga 1680

catagaactc cacgacgtgt ctcctattac tcggcacccc ctgcaagctc tcttcatctg 1740

ggccattctt cagaataaga aggaactctc caaagtcatt tgggagcaga ccaggggctg 1800

cactctggca gccctgggag ccagcaagct tctgaagact ctggccaaag tgaagaacga 1860

catcaatgct gctggggagt ccgaggagct ggctaatgag tacgagaccc gggctgttga 1920

gctgttcact gagtgttaca gcagcgatga agacttggca gaacagctgc tggtctattc 1980

ctgtgaagct tggggtggaa gcaactgtct ggagctggcg gtggaggcca cagaccagca 2040

tttcatcgcc cagcctgggg tccagaattt tctttctaag caatggtatg gagagatttc 2100

ccgagacacc aagaactgga agattatcct gtgtctgttt attataccct tggtgggctg 2160

tggctttgta tcatttagga agaaacctgt cgacaagcac aagaagctgc tttggtacta 2220

tgtggcgttc ttcacctccc ccttcgtggt cttctcctgg aatgtggtct tctacatcgc 2280

cttcctcctg ctgtttgcct acgtgctgct catggatttc cattcggtgc cacacccccc 2340

cgagctggtc ctgtactcgc tggtctttgt cctcttctgt gatgaagtga gacagtggta 2400

cgtaaatggg gtgaattatt ttactgacct gtggaatgtg atggacacgc tggggctttt 2460

ttacttcata gcaggaattg tatttcggct ccactcttct aataaaagct ctttgtattc 2520

tggacgagtc attttctgtc tggactacat tattttcact ctaagattga tccacatttt 2580

tactgtaagc agaaacttag gacccaagat tataatgctg cagaggatgc tgatcgatgt 2640

gttcttcttc ctgttcctct ttgcggtgtg gatggtggcc tttggcgtgg ccaggcaagg 2700

gatccttagg cagaatgagc agcgctggag gtggatattc cgttcggtca tctacgagcc 2760

ctacctggcc atgttcggcc aggtgcccag tgacgtggat ggtaccacgt atgactttgc 2820

ccactgcacc ttcactggga atgagtccaa gccactgtgt gtggagctgg atgagcacaa 2880

cctgccccgg ttccccgagt ggatcaccat ccccctggtg tgcatctaca tgttatccac 2940

caacatcctg ctggtcaacc tgctggtcgc catgtttggc tacacggtgg gcaccgtcca 3000

ggagaacaat gaccaggtct ggaagttcca gaggtacttc ctggtgcagg agtactgcag 3060

ccgcctcaat atccccttcc ccttcatcgt cttcgcttac ttctacatgg tggtgaagaa 3120

gtgcttcaag tgttgctgca aggagaaaaa catggagtct tctgtctgct gtttcaaaaa 3180

tgaagacaat gagactctgg catgggaggg tgtcatgaag gaaaactacc ttgtcaagat 3240

caacacaaaa gccaacgaca cctcagagga aatgaggcat cgatttagac aactggatac 3300
aaagcttaat gatctcaagg gtcttctgaa agagattgct aataaaatca aataaaactg 3360
tatgaactct aatggagaaa aatctaatta tagcaagatc atattaagga atgctgatga 3420
acaattttgc tatcgactac taaatgagag attttcagac ccctgggtac atggtggatg 3480
attttaaatc accctagtgt gctgagacct tgagaataaa gtgtgtgatt ggtttcatac 3540
ttgaagacgg atataaagga agaatatttc ctttatgtgt ttctccagaa tggtgcctgt 3600
ttctctctgt gtctcaatgc ctgggactgg aggttgatag tttaagtgtg ttcttaccgc 3660
ctcctttttc ctttaatctt atttttgatg aacacatata taggagaaca tctatcctat 3720
gaataagaac ctggtcatgc tttactcctg tattgttatt ttgttcattt ccaattgatt 3780
ctctactttt cccttttttg tattatgtga ctaattagtt ggcatattgt taaaagtctc 3840
tcaaattagg ccagattcta aaacatgctg cagcaagagg accccgctct cttcaggaaa 3900
agtgttttca tttctcagga tgcttcttac ctgtcagagg aggtgacaag gcagtctctt 3960
gctctcttgg actcaccagg ctcctattga aggaaccacc cccattccta aatatgtgaa 4020
aagtcgccca aaatgcaacc ttgaaaggca ctactgactt tgttcttatt ggatactcct 4080
cttattattt ttccattaaa aataatagct ggctattata gaaaatttag accatacaga 4140
gatgtagaaa gaacataaat tgtccccatt accttaaggt aatcactgct aacaatttct 4200
ggatggtttt tcaagtctat tttttttcta tgtatgtctc aattctcttt caaattttta 4260
cagaatgtta tcatactaca tatatacttt ttatgtaagc tttttcactt agtattttat 4320
caaatatgtt tttattatat tcatagcctt cttaaacatt atatcaataa ttgcataata 4380
ggcaacctct agcgattacc ataattttgc tcattgaagg ctatctccag ttgatcattg 4440
ggatgagcat ctttgtgcat gaatcctatt gctgtatttg ggaaaatttt ccaaggttag 4500
attccaataa atatctattt attattaaat attaaaatat ctatttatta ttaaaaccat 4560
ttataaggct ttttcataaa tgtatagcaa ataggaatta ttaacttgag cataagatat 4620
gagatacatg aacctgaact attaaaataa aatattatat ttaaccctta gtttaagaag 4680
aagtcaatat gcttatttaa atattatgga tggtgggcag atcacttgag gtcaggagtt 4740
cgagaccagc ctggccaaca tggcaaaacc acatctctac taaaaataaa aaaattagct 4800
gggtgtggtg gtgcactcct gtaatcccag ctactcagaa ggctgaggta caagaattgc 4860
tggaacctgg gaggcggagg ttgcagtgaa ccaagattgc accactgcac tccagccggg 4920
gtgacagagt gagactccga ctgaaaataa ataaataaat aaataaataa ataaataaat 4980
attatggatg gtgaagggaa tggtatagaa ttggagagat tatcttactg aacacctgta 5040
gtcccagctt tctctggaag tggtcgtatt tgagcaggat gtgcacaagg caattgaaat 5100

gcccataatt agtttctcag ctttgaatac actataaact cactggctga aggaggaaat 5160

tttagaagga agctactaaa agatctaatt tgaaaaacta caaaagcatt aactaaaaaa 5220

gtttattttc cttttgtctg ggcagtagtg aaaataacta ctcacaacat tcactatgtt 5280

tgcaaggaat taacacaaat aaaagatgcc tttttactta aacaccaaga cagaaaactt 5340

gcccaatact gagaagcaac ttgcattaga gagggaactg ttaaatgttt tcaacccagt 5400

tcatctggtg gatgtttttg caggttactc tgagaatttt gcttatgaaa aatcattatt 5460

tttagtgtag ttcacaataa tgtattgaac atacttctaa tcaaaggtgc tatgtccttg 5520

tgtatggtac taaatgtgtc ctgtgtactt ttgcacaact gagaatcctg cagcttggtt 5580

taatgagtgt gttcatgaaa taaataatgg aggaattgtc a 5621

<210> 2
<211> 1104
<212> PRT
<213> Homo sapiens

<400> 2

Met Ser Phe Arg Ala Ala Arg Leu Ser Met Arg Asn Arg Arg Asn Asp
1               5                   10                  15

Thr Leu Asp Ser Thr Arg Thr Leu Tyr Ser Ser Ala Ser Arg Ser Thr
            20                  25                  30

Asp Leu Ser Tyr Ser Glu Ser Asp Leu Val Asn Phe Ile Gln Ala Asn
        35                  40                  45

Phe Lys Lys Arg Glu Cys Val Phe Phe Thr Lys Asp Ser Lys Ala Thr
    50                  55                  60

Glu Asn Val Cys Lys Cys Gly Tyr Ala Gln Ser Gln His Met Glu Gly
65                  70                  75                  80

Thr Gln Ile Asn Gln Ser Glu Lys Trp Asn Tyr Lys Lys His Thr Lys
                85                  90                  95

Glu Phe Pro Thr Asp Ala Phe Gly Asp Ile Gln Phe Glu Thr Leu Gly
            100                 105                 110

Lys Lys Gly Lys Tyr Ile Arg Leu Ser Cys Asp Thr Asp Ala Glu Ile
        115                 120                 125

Leu Tyr Glu Leu Leu Thr Gln His Trp His Leu Lys Thr Pro Asn Leu
    130                 135                 140

Val Ile Ser Val Thr Gly Gly Ala Lys Asn Phe Ala Leu Lys Pro Arg

145                 150                 155                 160

Met Arg Lys Ile Phe Ser Arg Leu Ile Tyr Ile Ala Gln Ser Lys Gly
                165                 170                 175

Ala Trp Ile Leu Thr Gly Gly Thr His Tyr Gly Leu Met Lys Tyr Ile
            180                 185                 190

Gly Glu Val Val Arg Asp Asn Thr Ile Ser Arg Ser Ser Glu Glu Asn
        195                 200                 205

Ile Val Ala Ile Gly Ile Ala Ala Trp Gly Met Val Ser Asn Arg Asp
    210                 215                 220

Thr Leu Ile Arg Asn Cys Asp Ala Glu Gly Tyr Phe Leu Ala Gln Tyr
225                 230                 235                 240

Leu Met Asp Asp Phe Thr Arg Asp Pro Leu Tyr Ile Leu Asp Asn Asn
                245                 250                 255

His Thr His Leu Leu Leu Val Asp Asn Gly Cys His Gly His Pro Thr
            260                 265                 270

Val Glu Ala Lys Leu Arg Asn Gln Leu Glu Lys Tyr Ile Ser Glu Arg
        275                 280                 285

Thr Ile Gln Asp Ser Asn Tyr Gly Gly Lys Ile Pro Ile Val Cys Phe
    290                 295                 300

Ala Gln Gly Gly Gly Lys Glu Thr Leu Lys Ala Ile Asn Thr Ser Ile
305                 310                 315                 320

Lys Asn Lys Ile Pro Cys Val Val Val Glu Gly Ser Gly Gln Ile Ala
                325                 330                 335

Asp Val Ile Ala Ser Leu Val Glu Val Glu Asp Ala Leu Thr Ser Ser
            340                 345                 350

Ala Val Lys Glu Lys Leu Val Arg Phe Leu Pro Arg Thr Val Ser Arg
        355                 360                 365

Leu Pro Glu Glu Glu Thr Glu Ser Trp Ile Lys Trp Leu Lys Glu Ile
    370                 375                 380

Leu Glu Cys Ser His Leu Leu Thr Val Ile Lys Met Glu Glu Ala Gly
385                 390                 395                 400

Asp Glu Ile Val Ser Asn Ala Ile Ser Tyr Ala Leu Tyr Lys Ala Phe
                405                 410                 415

Ser Thr Ser Glu Gln Asp Lys Asp Asn Trp Asn Gly Gln Leu Lys Leu
            420                 425                 430

Leu Leu Glu Trp Asn Gln Leu Asp Leu Ala Asn Asp Glu Ile Phe Thr
        435                 440                 445

Asn Asp Arg Arg Trp Glu Ser Ala Asp Leu Gln Glu Val Met Phe Thr
    450                 455                 460

Ala Leu Ile Lys Asp Arg Pro Lys Phe Val Arg Leu Phe Leu Glu Asn
465                 470                 475                 480

Gly Leu Asn Leu Arg Lys Phe Leu Thr His Asp Val Leu Thr Glu Leu
                485                 490                 495

Phe Ser Asn His Phe Ser Thr Leu Val Tyr Arg Asn Leu Gln Ile Ala
            500                 505                 510

Lys Asn Ser Tyr Asn Asp Ala Leu Leu Thr Phe Val Trp Lys Leu Val
        515                 520                 525

Ala Asn Phe Arg Arg Gly Phe Arg Lys Glu Asp Arg Asn Gly Arg Asp
    530                 535                 540

Glu Met Asp Ile Glu Leu His Asp Val Ser Pro Ile Thr Arg His Pro
545                 550                 555                 560

Leu Gln Ala Leu Phe Ile Trp Ala Ile Leu Gln Asn Lys Lys Glu Leu
                565                 570                 575

Ser Lys Val Ile Trp Glu Gln Thr Arg Gly Cys Thr Leu Ala Ala Leu
            580                 585                 590

Gly Ala Ser Lys Leu Leu Lys Thr Leu Ala Lys Val Lys Asn Asp Ile
        595                 600                 605

Asn Ala Ala Gly Glu Ser Glu Glu Leu Ala Asn Glu Tyr Glu Thr Arg
    610                 615                 620

Ala Val Glu Leu Phe Thr Glu Cys Tyr Ser Ser Asp Glu Asp Leu Ala
625                 630                 635                 640

Glu Gln Leu Leu Val Tyr Ser Cys Glu Ala Trp Gly Gly Ser Asn Cys
                645                 650                 655

Leu Glu Leu Ala Val Glu Ala Thr Asp Gln His Phe Ile Ala Gln Pro
            660                 665                 670

Gly Val Gln Asn Phe Leu Ser Lys Gln Trp Tyr Gly Glu Ile Ser Arg
        675                 680                 685

Asp Thr Lys Asn Trp Lys Ile Ile Leu Cys Leu Phe Ile Ile Pro Leu
    690                 695                 700

Val Gly Cys Gly Phe Val Ser Phe Arg Lys Lys Pro Val Asp Lys His
705                 710                 715                 720

Lys Lys Leu Leu Trp Tyr Tyr Val Ala Phe Phe Thr Ser Pro Phe Val
                725                 730                 735

Val Phe Ser Trp Asn Val Val Phe Tyr Ile Ala Phe Leu Leu Leu Phe
            740                 745                 750

Ala Tyr Val Leu Leu Met Asp Phe His Ser Val Pro His Pro Pro Glu
        755                 760                 765

Leu Val Leu Tyr Ser Leu Val Phe Val Leu Phe Cys Asp Glu Val Arg
    770                 775                 780

Gln Trp Tyr Val Asn Gly Val Asn Tyr Phe Thr Asp Leu Trp Asn Val
785                 790                 795                 800

Met Asp Thr Leu Gly Leu Phe Tyr Phe Ile Ala Gly Ile Val Phe Arg
                805                 810                 815

Leu His Ser Ser Asn Lys Ser Ser Leu Tyr Ser Gly Arg Val Ile Phe
            820                 825                 830

Cys Leu Asp Tyr Ile Ile Phe Thr Leu Arg Leu Ile His Ile Phe Thr
        835                 840                 845

Val Ser Arg Asn Leu Gly Pro Lys Ile Ile Met Leu Gln Arg Met Leu
    850                 855                 860

Ile Asp Val Phe Phe Phe Leu Phe Leu Phe Ala Val Trp Met Val Ala
865                 870                 875                 880

Phe Gly Val Ala Arg Gln Gly Ile Leu Arg Gln Asn Glu Gln Arg Trp
                885                 890                 895

Arg Trp Ile Phe Arg Ser Val Ile Tyr Glu Pro Tyr Leu Ala Met Phe
            900                 905                 910

Gly Gln Val Pro Ser Asp Val Asp Gly Thr Thr Tyr Asp Phe Ala His
        915                 920                 925

Cys Thr Phe Thr Gly Asn Glu Ser Lys Pro Leu Cys Val Glu Leu Asp
    930                 935                 940

Glu His Asn Leu Pro Arg Phe Pro Glu Trp Ile Thr Ile Pro Leu Val
945                 950                 955                 960

Cys Ile Tyr Met Leu Ser Thr Asn Ile Leu Leu Val Asn Leu Leu Val
                965                 970                 975

Ala Met Phe Gly Tyr Thr Val Gly Thr Val Gln Glu Asn Asn Asp Gln
            980                 985                 990

Val Trp Lys Phe Gln Arg Tyr Phe Leu Val Gln Glu Tyr Cys Ser Arg
        995                 1000                1005

Leu Asn Ile Pro Phe Pro Phe Ile Val Phe Ala Tyr Phe Tyr Met
    1010                1015                1020

Val Val Lys Lys Cys Phe Lys Cys Cys Cys Lys Glu Lys Asn Met
    1025                1030                1035

Glu Ser Ser Val Cys Cys Phe Lys Asn Glu Asp Asn Glu Thr Leu
    1040                1045                1050

Ala Trp Glu Gly Val Met Lys Glu Asn Tyr Leu Val Lys Ile Asn
    1055                1060                1065

Thr Lys Ala Asn Asp Thr Ser Glu Glu Met Arg His Arg Phe Arg
    1070                1075                1080

Gln Leu Asp Thr Lys Leu Asn Asp Leu Lys Gly Leu Leu Lys Glu
    1085                1090                1095

Ile Ala Asn Lys Ile Lys
    1100

## Beschreibung

Die Erfindung betrifft neuartige Modulatoren des Kälte-Menthol-Rezeptors TRPM8, Verfahren zur Modulation des TRPM8-Rezeptors unter Verwendung dieser Modulatoren; die Verwendung der Modulatoren zur Induktion von Kältegefühl; sowie die unter Verwendung dieser Modulatoren hergestellten Gegenstände und Mittel.

### Hintergrund der Erfindung

Der Kälte-Menthol-Rezeptor TRPM8 (auch bezeichnet als Cold-Membrane Receptor (CMR)1) gehört zur Familie der "Transient Receptor Potential Ion Channels", wird spezifisch in einer speziellen Gruppe von Neuronen exprimiert und bildet in der Zellmembran Poren aus (jeweils 4 Einheiten lagern sich dabei zu einem Tetramer zusammen), die selektiv Ca²⁺ Ionen passieren lassen. Das Protein weist 6 Transmembrandomänen auf und einen cytoplasmatischen C- sowie N-Terminus. Durch niedrige Temperaturen (bevorzugt 10-25°C) wird dieser Rezeptor stimuliert, es kommt zu einer Signaltransduktion, die vom Nervensystem als Kältegefühl interpretiert wird. Der Rezeptor ist erstmals 2002 als Kälterezeptor in mehreren Publikationen beschrieben worden (Peier AM et al, .A TRP channel that senses cold stimuli and menthol.Cell. 2002 Mar 8;108(5):705-15; McKemy DD et al. Identification of a cold receptor reveals a general role for TRP channels in thermosensation. Nature. 2002 Mar 7;416(6876):52-8; Zuker CS. Neurobiology: a cool ion channel.Nature. 2002 Mar 7;416(6876):27-8)

Kühlende Verbindungen, wie z.B. Menthol, spielen bereits seit langem eine wichtige Rolle in der Geschmacks- und Riechstoffindustrie, um eine Assoziation mit Frische und Sauberkeit zu erzeugen. Für die Verbindung Menthol ist gezeigt worden, dass sie als ein natürlicher Modulator des Rezeptors TRPM8 wirkt (McKemy D.D., Molecular Pain 1, 2005, 16; McKemy D.D., Nature 416, 2002, 52-58; Peier A.M., Cell 108, 2002, 705-715; Dhaka A., Annu. Rev. Neurosci. 29, 2006, 135-161). Durch Applikation von Menthol wird TRPM8 aktiviert, wodurch ein Ca²⁺-Einstrom in die Kälte-sensitiven Neuronen bewirkt wird. Das dadurch erzeugte elektrische Signal wird schließlich als Kältegefühl wahrgenommen. Überhöhte Menthol-Konzentrationen führen zu Irritation und einer anästhetischen Wirkung. Darüber hinaus sind in verschiedenen Publikationen Mentholderivate mit ähnlicher Wirkung beschrieben worden (British Patent 1971#1315761; Watson H.R., J. Soc. Cosmet. Chem. 29, 1978, 185-200; Furrer S.M., Chem. Percept. 1, 2008, 119-126). Es gibt auch vereinzelte, strukturell mit Menthol nicht verwandte Verbindungen, die eine signifikante TRPM8 Modulation bewirken, wie z. B. Icilin (Wei E.T., J. Pharm. Pharmacol. 35, 1983, 110-112; WO 2004/026840), WS-23 oder in der Patentanmeldung WO 2007/019719 aufgeführte Verbindungen.

Weitere Wirkungen von Substanzen, die den TRPM8 Rezeptor bzw. seine Insektenanaloga modulieren, sind eine Repellentwirkung auf Insekten (WO 2002/015692; WO 2004/000023, US 2004/0028714), sowie Aktivität in der Antitumortherapie (z.B. eine Beeinflussung von Prostatatumoren), Aktivität bei der Behandlung von inflammatorischen Schmerzen / Hyperalgesie und eine Wirkung als TRPM8 Antagonisten in der Therapie von Blasensyndrom bzw. überaktiver Blase (Beck B. Cell Calcium, 41, 2007, 285-294; Levine J.D. Biochim. Biophys. Acta, Mol. Basis Dis. 1772, 2007, 989-1003; Mukerji G., BMC Urology 6, 2006, 6; US 2003/0207904; US 2005/6893626, Dissertation Behrendt H.J. 2004, Universität Bochum; Lashinger E.S.R. Am. J. Physiol. Renal Physiol. Am J Physiol Renal Physiol. 2008 Jun 18. [Epub ahead of print]; PMID: 18562636).

Viele der bisher gefundenen Modulatoren von TRPM8 weisen jedoch Mängel in Bezug auf Wirkstärke, Wirkdauer, Haut-/Schleimhautreizung, Geruch, Geschmack, Löslichkeit und /oder Flüchtigkeit auf.

### Kurzfassung der Erfindung

Aufgabe der vorliegenden Erfindung war es daher, neue Substanzen zu identifizieren, die zu einer Modulation des TRPM8 Rezeptors führen, welche als Alternativen zu den bisher bekannten Modulatoren einsetzbar sind. Solche Verbindungen sollten sich insbesondere auch für Anwendungen im Bereich Kosmetik (z.B. hair care, skin care, oral care), Ernährung (feed/food), Textil, OTC-Produkte (z.B. Brandsalbe), Pharmaka (z.B. Tumorbehandlung, Blasenschwäche), Verpackungen oder als Insektizid bzw. Repellent eignen.

### Figurenbeschreibung:

Figur 1 zeigt (a) die mRNA-Sequenz bzw. (b) die davon abgeleitete Aminosäuresequenz des hTRPM8 Rezeptors gemäß Sequenzdatenbank-Eintrag NM 024080.
Figur 2 zeigt die Vektorkarte des mit hTRPM8 kodierenden Plasmids plnd_M8, welches zur Transfektion der HEK293 Zellen verwendet wurde.

### Detaillierte Beschreibung der Erfindung:

### 1. Definition allgemeiner Begriffe:

In der Literatur gibt es verschiedene Synonyme für "TRPM8": TRPP8, LTRPC6, CMR1, MGC2849, transient receptor potential cation channel subfamily M member 8. Im Sinne der vorliegenden Erfindung sind alle Bezeichnungen mit umfasst. Mit umfasst sind auch alle funktionalen Modifikationen des Rezeptors, wie insbesondere Splicevarianten, Isoformen, wie z.B. TRPM8 CRA_a, TRPM8 CRA_b und alle analogen Rezeptoren aus verschiedenen Organismen, wie Mensch, Maus, Ratte. Die Nukleotid- bzw. Aminosäuresequenzen der verschiedenen Rezeptoren sind an sich bekannt und in Sequenzdatenbanken hinterlegt. So ist z.B. die Sequenzinformation für hTRPM8 unter der Nummer NM_024080 eingetragen.

Ein "Modulator" im Sinne der Erfindung stellt eine Verbindung dar, die als Agonist und/oder Antagonist des TRPM8-Rezeptors in vivo und/oder in vitro wirken kann.

Geeignete Modulatoren können dabei entweder nur als Antagonist oder Agonist oder sowohl als Antagonist als auch als Agonist agieren. Dabei können sich insbesondere eine agonistische oder eine antagonistische Wirkung in Abhängigkeit von der jeweiligen gewählten Modulatorkonzentration einstellen.

Ein "Agonist" ist dabei eine Verbindung, welche eine Aktivierung des TRPM8-Rezeptors vermittelt, also einen Ca²⁺ -Einstrom in die kälte-sensitiven Neuronen induziert und damit ein Kältegefühl vermittelt. Ein "Antagonist" ist dagegen eine Verbindung, welche dieser Aktivierung des TRPM8-Rezeptors entgegenwirken kann.

Die erfindungsgemäßen Mediatoren können ihre Wirkung dadurch ausüben, dass sie reversibel oder irreversibel, spezifisch oder unspezifisch an ein TRPM8 Rezeptormolekül binden. Gewöhnlich erfolgt die Bindung nicht-kovalent über ionische und/oder nichtionische, wie z.B. hydrophobe, Wechselwirkungen mit dem Rezeptormolekül. "Spezifisch" umfasst dabei sowohl ausschließliche Wechselwirkung mit einem oder mehreren verschiedenen TRPM8 Rezeptormolekülen (wie z.B. TRPM8-Molekülen verschiedenen Ursprungs oder verschiedenen Isoformen). "Unspezifisch" ist dagegen eine Wechselwirkung des Modulators mit mehreren verschiedenen Rezeptormolekülen unterschiedlicher Funktion und/oder Sequenz, wobei sich jedoch als Folge eine gewünschte agonistische und/oder antagonistische Modulation (wie oben beschrieben) des TRPM8 Rezeptors feststellen lässt.

### 2. Bevorzugte Ausführungsformen

Ein erster Gegenstand der Erfindung betrifft ein Verfahren zur in-vitro oder in-vivo Modulation des Kälte-Menthol-Rezeptors TRPM8, insbesondere des humanen TRPM8 Rezeptors, wobei man den Rezeptor mit wenigstens einer Verbindung in Kontakt bringt, die ausgewählt ist unter mehrkernigen organischen Verbindungen, welche in einem zellulären Aktivitätstest, insbesondere unter Standardbedingungen, unter Verwendung von Zellen, welche den humanen TRPM8-Rezeptor rekombinant exprimieren, die Permeabilität dieser Zellen für Ca²⁺ Ionen modulieren.

Unter "Standardbedingungen" versteht man in diesem Zusammenhang einen Aktivitätstest, durchgeführt mit HEK293-Zellen, welche transformiert wurden mit humanem TRPM8 und beladen sind mit Calcium-sensitivem Farbstoff (wie z.B. Fluo-4AM, d.h. Fluo-4-Acetoxymethylester), anschließende Zugabe der Testverbindung und Nachweis der Farbänderung, wobei Versuchsdurchführung bei 37 °C erfolgt; wie z.B. beschrieben in Beispiel 3, unten, oder bei Behrendt et al (2004) a.a.O).

Insbesondere umfasst dabei die modulierende Verbindung wenigstens zwei 4- bis 7-gliedrige Ringe, die unabhängig voneinander carbo- oder hetrerocyclisch, mono- oder polycyclisch sind, und wobei wenigstens zwei dieser Ringe gegebenenfalls kondensiert oder spiro-verknüpft sein können. Andere nichtlimitierende Beispiele geeigneter Ring-Verknüpfungen umfassen chemische Einfachbindungen zwischen Ringkohlenstoff- und/oder Ringheteroatomen, über 2 bis 6-gliedrige Kohlenstoffbrückengruppen, wobei einzelne C-Atome durch Heteroatome, wie N, O oder S, ersetzt sein können. Außerdem können die Ringgruppen und Brückengruppen ggf. Substituenten tragen, die ausgewählt sind unter Ketogruppen, -OH, -SH, -CN, -NO₂, -C₁₋₆-Alkyl, oder C₂₋₄-Alkenyl, wobei in den Alkyl- oder Alkenylgruppen ein oder mehrere H-Atome durch Halogen, wie F, Cl, Br oder J ersetzt sein können.

Carbocyclische Ringe umfassen dabei 4, 5, 6 oder 7 Kohlenstoffatome; heterocyclische Ringe umfassen neben den Ringkohlenstoffatomen 1 bis 3 gleiche oder verschiedene Ring-Heteroatome, wie O, N und S-Atome. Die Ringe können dabei unabhängig voneinander gesättigt, ein- oder mehrfach ungesättigt, wie z.B. aromatische Ringe, sein.

Die erfindungsgemäß eingesetzten Modulator wirken dabei auf die zelluläre Ca²⁺-lonen-Permeabilität agonistisch oder antagonistisch. Insbesondere ist der Modulator wenigstens eine Verbindung, ausgewählt unter Verbindungen der folgenden Formeln 1 bis 19 gemäß folgender Tabelle 1

**Tabelle 1: Erfindungsgemäße Modulatoren**

| | |
|---|---|
| 1 | 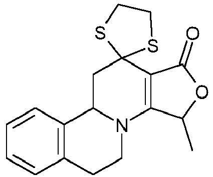 |
| 2 | 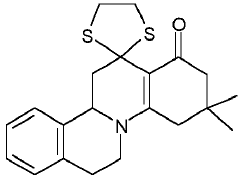 |
| 3 | 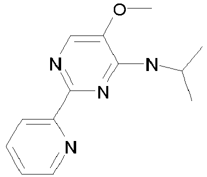 |
| 4 | 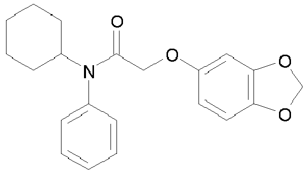 |
| 5 | 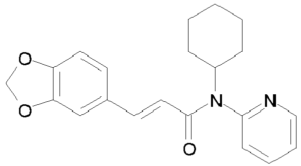 |
| 6 | 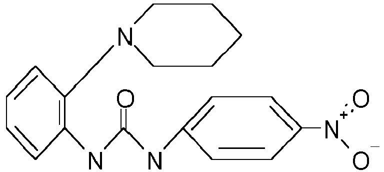 |
| 7 | 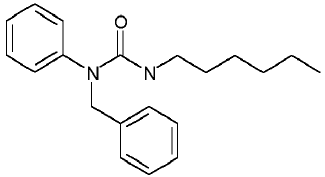 |
| 8 | 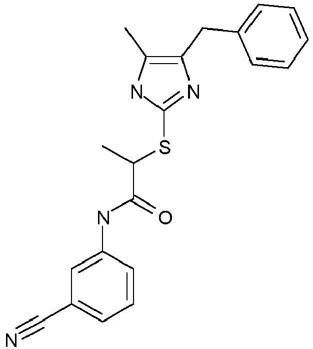 |
| 9 | 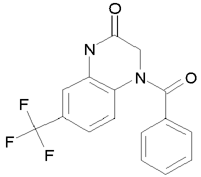 |
| 10 | 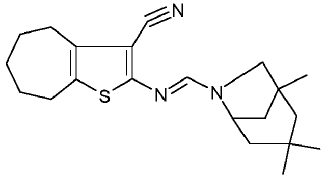 |
| 11 | 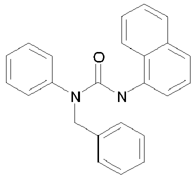 |
| 12 | 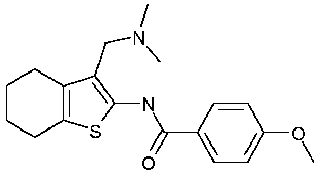 |
| 13 | 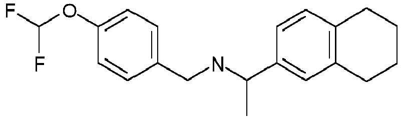 |
| 14 | 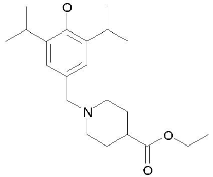 |
| 15 | 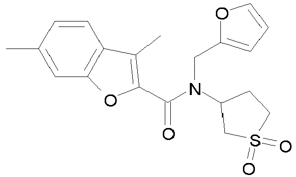 |
| 16 | 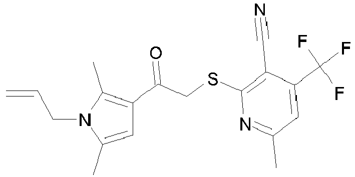 |
| 17 | 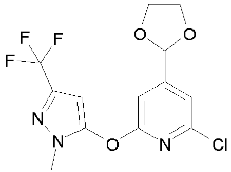 |
| 18 | 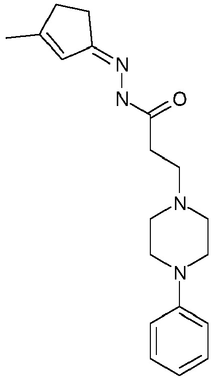 |
| 19 | 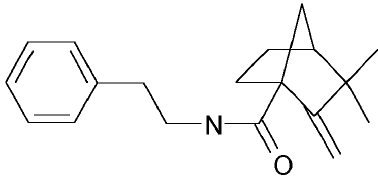 |

wobei die Verbindung in chemisch reiner oder angereicherter Form, als einzelnes Stereoisomer oder in Form von Stereoisomeren-Gemischen vorliegen kann. Weiterhin können die Verbindungen ungeladen oder in Form ihrer Salze, wie z.B. als Säureadditionssalz, vorliegen. Funktionelle Gruppen können ggf. durch äquivalente chemische Gruppen ersetzt sein; Fluoratome können so z.B. durch andere Halogenatome , wie Cl, Br oder J, ersetzt sein, Sauerstoffgruppen (wie z.B. Ethergruppen) können durch entsprechende Schwefelgruppen ersetzt sein und umgekehrt; Ketogruppen können durch entsprechende Thionylgruppen ersetzt sein. Die oben genannten Verbindungen sind an sich bekannte chemische Substanzen, die entweder käuflich erhältlich oder unter Anwendung üblicher Methoden der organischen Synthese zugänglich sind.

So sind beispielsweise bekannt:
Verbindung 1 unter CAS Nummer: 99602-94-5 (3R-cis-Form)
Verbindung 2 unter CAS Nummer: 165753-08-2
Verbindung 3 unter CAS Nummer: 338771-57-6
Verbindung 4 unter CAS Nummer: 878942-21-3
Verbindung 5 unter CAS Nummer: 748783-13-3

Die abgewandelten Formen oder Derivate werden auch als funktionale Analoga oder funktional äquivalente Verbindungen bezeichnet, wenn sie weiterhin die gewünschte biologische Aktivität (Rezeptor TRPM8 Modulation) zeigen.

Weiterhin sind besonders Derivate im Sinne der Erfindung mit umfasst, die eine Kopplung der konkret offenbarten Substanzen an feste Träger erlauben; eine große Auswahl an entsprechenden Linkern/Spacer-Gruppen ist dem Fachmann bekannt. Die Derivatisierung kann dabei vor der Kopplung an eine feste Phase oder erst durch die Kopplung erfolgen.

Ein weiterer Gegenstand der Erfindung betrifft die Verwendung eines Modulators, insbesondere Agonisten, für den TRPM8-Rezeptor, wobei der Modulator wie oben definiert ist, zur Induktion von Kältegefühl, insbesondere topisch, d.h. cutan oder oral, bei Mensch und/oder Tier. Eine "Induktion von Kältegefühl" liegt vor, wenn die Verbindung im oben beschriebenen zellulären Aktivitätstest einen agonistischen Effekt auf hTRPM8 zeigt.

Ein weiterer Gegenstand der Erfindung betrifft die Verwendung eines Modulators für den TRPM8-Rezeptor, wobei der Modulator wie oben definiert ist, als aktiven Bestandteil eines pharmazeutischen Mittels.

Ein weiterer Gegenstand der Erfindung betrifft die Verwendung eines Modulators für den TRPM8-Rezeptor, wobei der Modulator wie oben definiert ist, zur Behandlung von Prostatakarzinomen, zur Behandlung von Blasenschwäche oder in der Schmerztherapie.

Ein weiterer Gegenstand der Erfindung betrifft die Verwendung eines Modulators für den TRPM8-Rezeptor, wobei der Modulator wie oben definiert ist, als Insekten-Repellent oder Insektizid.

Ein weiterer Gegenstand der Erfindung betrifft die Verwendung eines Modulators für den TRPM8-Rezeptor, wobei der Modulator wie oben definiert ist, zur Induktion eines Kältegefühls bei Verpackungen (z.B. aus Papier oder Kunststoff in verschiedensten Verarbeitungsformen (wie z.B. Fasern, Geweben, Formteilen), wobei sich das Kältegefühl insbesondere bei Kontakt mit dem Verpackungsmaterial bemerkbar macht. Dabei können die Substanzen in unterschiedlichster Art mit dem Verpackungsmaterial assoziiert sein: z.B. durch Spincoating, Aufdrucken, in Form von Mikroverkapselung, direkte Einarbeitung in das Verpackungsmaterial (z.B. extrudieren), kovalente Kopplung von geeigneten Derivaten der Modulatoren (über geeignete Spacer/Linker-Gruppen, mit deren Hilfe das Molekül reversibel oder irreversibel an das Verpackungsmaterial gebunden wird). Geeignete Arbeitsweisen sind dem Fachmann bekannt.

Ein weiterer Gegenstand der Erfindung betrifft die Verwendung eines Modulators für den TRPM8-Rezeptor, wobei der Modulator wie oben definiert ist, zur Induktion eines Kältegefühls bei Textilien. Dabei können die Substanzen in unterschiedlichster Art mit dem Textil assoziiert sein: z.B. durch Spincoating, Aufdrucken, in Form von Mikroverkapselung, direkte Einarbeitung in das Textilmaterial (z.B. extrudieren), kovalente Kopplung von geeigneten Derivaten der Modulatoren (über geeignete Spacer/Linker-Gruppen, mit deren Hilfe das Molekül reversibel oder irreversibel an das Verpackungsmaterial gebunden wird). Geeignete Arbeitsweisen sind dem Fachmann bekannt.

Ein weiterer Gegenstand der Erfindung betrifft Stoffe per se gemäß obiger Definition zur Verwendung als Mediator, insbesondere Agonisten und/oder Antagonisten, des TRPM8 Rezeptors.
Ein weiterer Gegenstand der Erfindung betrifft Mittel, enthaltend wenigstens eine Verbindung nach obiger Definition. Insbesondere sind solche Mittel ausgewählt unter
a) pharmazeutischen Mitteln, wie Antitumormittel, Mittel zur Behandlung von Erkrankungen der Blase, Schmerzmittel;
b) Nahrungsmitteln, wie Eis, Mousse, Creme, Getränke, Süßwaren,
c) Mundpflegemitteln, wie Zahnpasta, Mundwasser, Kaugummi, Atemerfrischungsmittel
d) Haut-, oder Haarpflegemitteln, wie Sonnencreme, Sonnenbrandcreme, Lotionen, Shampoos, Pflaster, Mundwasser, Lotionen, Rasiercreme, Conditioner, Gesichtsreiniger, Seifen, Badeöle und Badeschäume, Antitranspirationsmittel, desodorierende Mittel,
e) Insektenrepellents, Insektiziden.

Derartige Mittel enthalten neben für das jeweilige Mittel jeweils üblichen Bestandteilen eine wirksame Menge wenigstens eines erfindungsgemäßen Modulators. "Wirksam" bedeutet in diesem Zusammenhang eine Konzentration des Modulators, die ausreicht, um bei Applikation des Mittels (z.B. Auftragung auf die Haut) den gewünschten Effekt, wie z.B. pharmakologischen Effekt, oder sensorischen Effekt wie z.B. den olfaktorischen Kälteeffekt zu vermitteln.

Gegebenenfalls können die erfindungsgemäßen Verbindungen mit weiteren bekannten Wirkstoffen, insbesondere auch solchen mit vergleichbarer Wirkung, kombiniert werden. Beispielsweise können diese mit bekannten kühlenden Verbindungen, wie z.B. Menthol, Menthon, N-Ethyl-p-menthancarboxamid (WS-3), N-2,3-Trimethyl-2-isopropylbutanamid (WS-23), Menthyllactat (Frescolat® ML), Menthonglycerinacetal (Frescolat® MGA), Mono-menthylsuccinat (Physcool ®), Mono-menthyl glutarat, O-Menthyl Glycerin, Menthyl-N,N-dimethylsuccinamat kombiniert werden.

Gegenstand der Erfindung sind weiterhin Textilprodukte, wie z.B. Hemden, Hosen, Socken, Handtücher, ausgerüstet (insbesondere oberflächlich) mit wenigstens einer Verbindung nach obiger Definition.

Gegenstand der Erfindung sind weiterhin Verpackungsmaterialien, welche mit wenigstens einer Verbindung nach obiger Definition assoziiert sind.

Die Erfindung wird nun anhand folgender nicht-limitierender Ausführungsbeispiele beschrieben.

### Experimenteller Teil:

### Beispiel 1 - Klonierung von humanem TRPM8

Ausgangspunkt für die Klonierung des humanen TRPM8 Rezeptors ist eine LnCaP cDNA-Bank. Diese ist beispielsweise kommerziell erhältlich (z. B. Firma BioChain, Hayward, USA) oder aus der androgensensitiven humanen Prostata-Adenokarzinomzelllinie LnCaP (z.B. ATCC, CRL1740 oder ECACC, 89110211) unter Verwendung von Standardkits herstellbar.

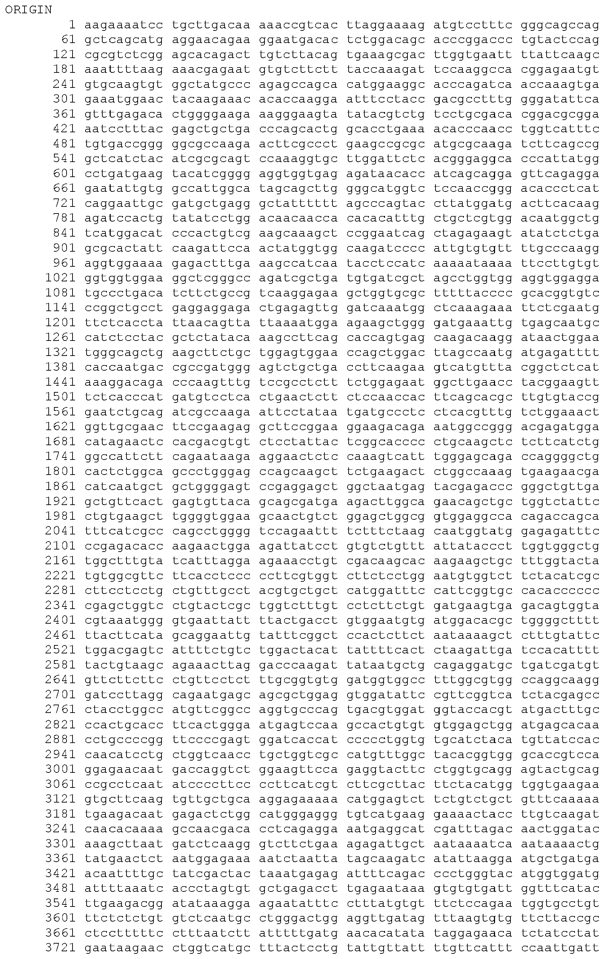

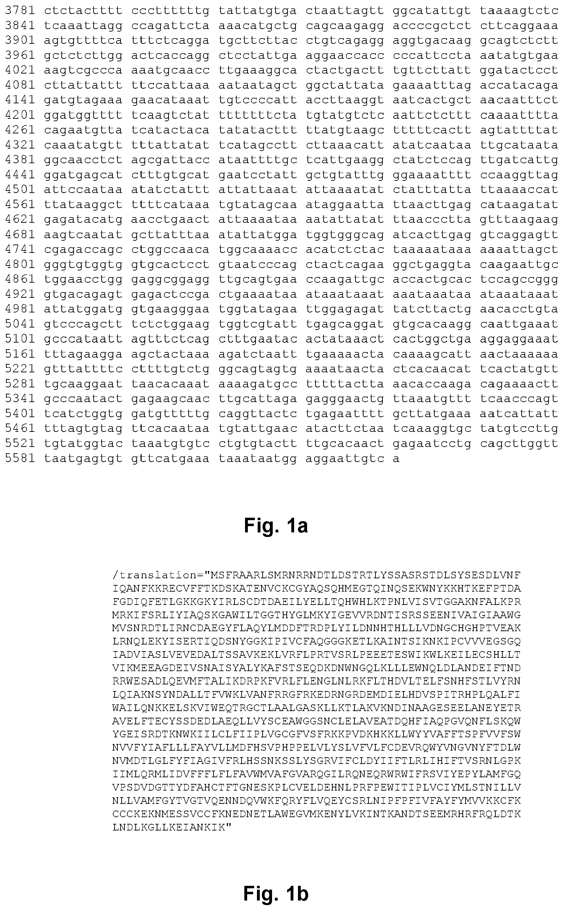

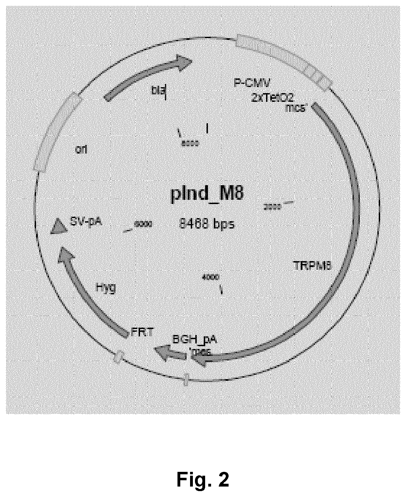

Die kodierende TRPM8 Sequenz (vgl Fig.1A; sowie **http://www.ncbi.nlm.nih.gov/entrez/viewer.fcgi?db=nuccore&id=109689694)** kann unter Verwendung von Standardmethoden PCR-amplifiziert und kloniert werden. Das so isolierte humane TRPM8 Gen wurde zur Herstellung des Plasmids plnd_M8 verwendet, dessen Konstruktion durch die Plasmidkarte gemäß Figur 2 veranschaulicht wird.

Alternativ dazu kann das TRPM8 Gen auch synthetisch hergestellt werden.

### Beispiel 2 - Generierung der HEK293-Testzellen

Als Testzellsystem wurde mit der humanen TRPM8 DNA (vgl obiges Plasmid plnd-M8) stabil transfizierte HEK293 Zelllinie hergestellt. Bevorzugt wird dabei HEK293 die über das eingebrachte Plasmid die Möglichkeit zur Induktion der TRPM8-Expression mittels Tetrazyklin bietet.

Methoden zur Herstellung geeigneter Testzellsysteme sind dem Fachmann bekannt. So kann man die Herstellung der erfindungsgemäß verwendeten Zellen den Angaben in Behrendt H.J. et al.,, Br. J. Pharmacol. 141, 2004, 737-745 oder der Dissertation von Behrendt "Vergleichende funktionale Untersuchungen des Hitze-Capsaicin-Rezeptors (TRPV1) und des Kälte-Menthol-Rezeptors (TRPM8) in rekombinanten und nativen Zellssystemen", zugänglich unter http://www-brs.ub.ruhr-uni-bochum.de/netahtml/HSS/Diss/BehrendtHansJoerg/diss.pdf entnehmen. Auf die Offenbarung dieser Druckschriften wird ausdrücklich Bezug genommen.

### Beispiel 3- Assay auf TRPM8 Modulatoren

Es wird ein Test, vergleichbar mit dem bereits in der Literatur beschrieben Test von Behrendt H.J. et al., Br. J. Pharmacol. 141, 2004, 737-745, durchgeführt. Die Agonisierung bzw. Antagonisierung des Rezeptors kann mittels eines Ca²⁺-sensitiven Farbstoffs (z.B. FURA, Fluo-4 etc.) quantifiziert werden. Agonisten bewirken alleine eine Zunahme des Ca²⁺-Signals; Antagonisten bewirken in Gegenwart von z.B. Menthol eine Reduzierung des Ca²⁺-Signals (jeweils detektiert über den Farbstoff Fluo-4, der durch Ca²⁺ andere Fluoreszenzeigenschaften hat).

### a) Testablauf:

Zunächst wird in an sich bekannter Weise in Zellkulturflaschen eine frische Kultur transformierter HEK-Zellen hergestellt. Die Testzellen HEK293-TRPM8 werden mittels Trypsin von den Zellkulturflaschen abgelöst und 40.000 Zellen/well werden mit 100 µl Medium in 96-Lochplatten (Greiner # 655948 Poly-D-Lysin-beschichtet) ausgesät. Zur Induktion des Rezeptors TRPM8 wird dem Wachstumsmedium Tetrazyklin beigemischt (DMEM/HG, 10% FCS tetrazyklinfrei, 4 mM L-Glutamin, 15 µg/ml Blasticidin, 100 µg/ml Hygromycin B, 1 µg/ml Tetrazyklin). Am darauffolgenden Tag werden die Zellen mit Fluo-4AM Farbstoff beladen und der Test wird durchgeführt. Dazu wird wie folgt vorgegangen:
- Zugabe von je 100 µl/well Färbelösung Ca-4 Kit (RB 141, Molecular Devices) zu je 100 µl Medium (DMEM/HG, 10% FCS tetrazyklinfrei, 4 mM L-Glutamin, 15 µg/ml Blasticidin, 100 µg/ml Hygromycin B, 1 µg/ml Tetrazyklin)
- Inkubation im Brutschrank, 30 Minuten / 37°C / 5% CO₂, 30 Minuten / RT
- Vorbereitung der Testsubstanzen (unterschiedliche Konzentrationen in 200 µl HBSS-Puffer), sowie von Positivkontrollen (unterschiedliche Konzentrationen von Menthol, Icilin bzw. lonomycin in 200 µl HBSS-Puffer) und Negativkontrollen (nur 200 µl HBSS-Puffer)
- Zugabe der Testsubstanzen in Mengen von 50 µl/well und Messung der Fluoreszenzänderung (z.B. im Assaygerät FLIPR, Molecular Devices oder NovoStar, BMG) bei 485 nm Anregung, 520 nm Emission, und Auswertung der Wirkstärke der verschiedenen Substanzen/Konzentrationen und Bestimmung der EC50-Werte

Die Testsubstanzen werden in Triplikaten in Konzentrationen von 0,1 - 200 µM im Assay eingesetzt. Normalerweise werden die Verbindungen in DMSO-Lösungen bereitgehalten und für den Assay auf eine maximale DMSO-Konzentration von 2% herunterverdünnt.

### b) Testergebnis

Die ermittelten EC50-Werte erfindungsgemäßer Modulatoren sind in folgender Tabelle 2 zusammengefasst

**Tabelle 2: Aktivität von Testsubstanzen auf den human Rezeptor TRPM8**

| **#** | **Activität TRPM8 EC50** |
|---|---|
| 1 | 0,4 |
| 2 | 2 |
| 3 | 2 |
| 4 | 2,5 |
| 5 | 2,5 |
| 6 | 3,5 |
| 7 | 4 |
| 8 | 5 |
| 9 | 10 |
| 10 | 10 |
| 11 | 10 |
| 12 | 10 |
| 13 | 10 |
| 14 | 20 |
| 15 | 20 |
| 16 | 25 |
| 17 | 50 |
| 18 | 100 |
| 19 | 100 |

Die Auswertung zeigt überraschenderweise, dass erfindungsgemäß erstmalig Agomisten von TRPM8 bereitgestellt werden konnten, welche sich strukturell von bisher bekannten Agonisten, wie (-) Menthol, Icilin und anderen von Behrendt H.J. et al.,in Br. J. Pharmacol. 141, 2004, 737-745 (vgl dortige Tabelle 1) beschriebenen Modulatoren signifikant unterscheiden und zudem teilweise bessere Aktivitäten als (-) Menthol zeigen, bzw. vergleichbar stark wirken wie Icilin.

### Beispiel 4 - Herstellung von Mundwasser

Ein Mundwasser folgender Zusammensetzung wird hergestellt:

| | |
|---|---|
| Ethanol 95% | 177mL |
| Sorbit 70% | 250 g |
| TRPM8 Agonist gemäß von Tab.2 als 1% Lösung im Ethanol | 50mL |
| Pfefferminzöl, | 0.30 g |
| Methylsalicylat | 0.64 g |
| Eucalyptol | 0.922 g |
| Thymol | 0.639 g |
| Benzoesäure | 1.50 g |
| Pluronic ® F127 nichtionisches Tensid | 5.00 g |
| Natrium-Saccharin | 0.60 g |
| Natriumcitrat | 0.30 g |
| Zitronensäure | 0.10 g |
| Wasser q.s. 1 Liter | |

Zur Herstellung eines Mundwassers werden die oben beschriebenen Komponenten in den angegebenen Mengen miteinander vermischt.

Auf die Offenbarung der hierin zitierten Literaturstellen wird hiermit ausdrücklich Bezug genommen.

Weitere Ausführungsformen der Erfindung:
2. Verfahren zur in-vitro oder in-vivo Modulation des Kälte-Menthol-Rezeptors TRMP8, wobei man den Rezeptor mit wenigstens einer Verbindung in Kontakt bringt, die ausgewählt ist unter mehrkernigen organischen Verbindungen, welche in einem zellulären Aktivitätstest unter Verwendung von Zellen, welche den humanen TRPM8-Rezeptor rekombinant exprimieren, die Permeabilität dieser Zellen für Ca²⁺ Ionen modulieren.
3. Verfahren nach Ausführungsform 1, wobei die modulierende Verbindung wenigstens zwei 4- bis 7-gliedrige Ringe ausweist, die unabhängig voneinander carbo- oder hetrerocyclisch, mono- oder polycyclisch sind, und wobei wenigstens zwei dieser Ringe gegebenenfalls kondensiert sein können.
4. Verfahren nach Ausführungsform1 oder 2, wobei der Modulator auf die zelluläre Ca²⁺-Ionen-Permeabilität agonistisch oder antagonistisch wirkt.
5. Verfahren nach einem der vorhergehenden Ausführungsformen, wobei der Modulator eine Verbindung ist, ausgewählt unter Verbindungen der Formeln 1 bis 19

| | |
|---|---|
| 1 | 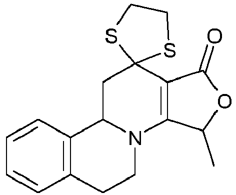 |
| 2 | 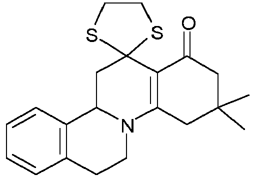 |
| 3 | 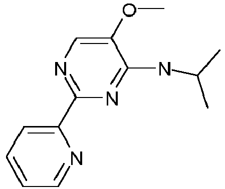 |
| 4 | 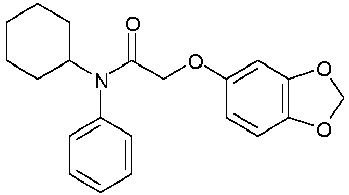 |
| 5 | 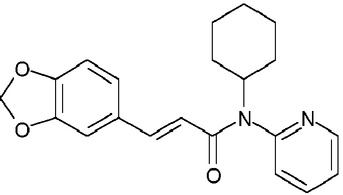 |
| 6 | 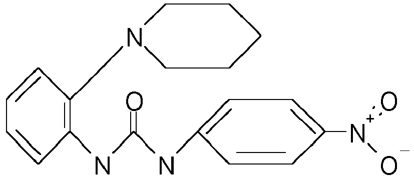 |
| 7 | 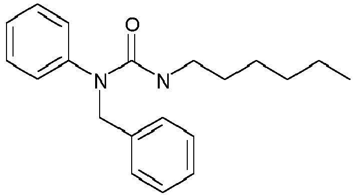 |
| 8 | 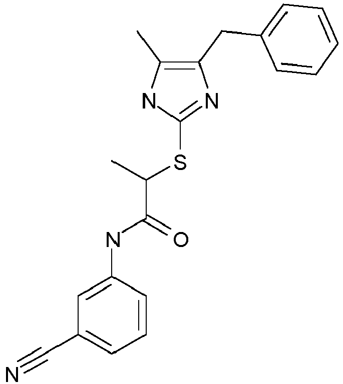 |
| 9 | 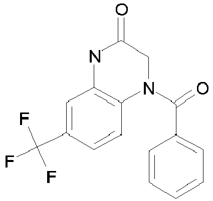 |
| 10 | 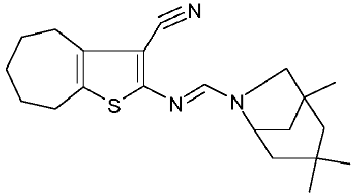 |
| 11 | 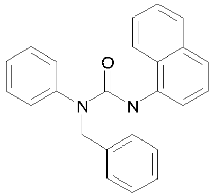 |
| 12 | 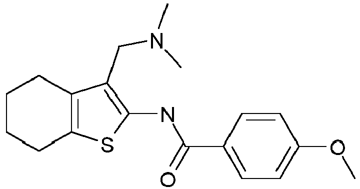 |
| 13 | 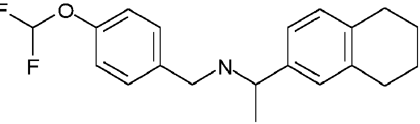 |
| 14 | 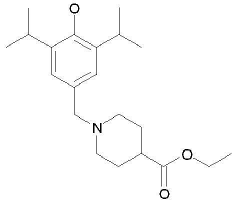 |
| 15 | 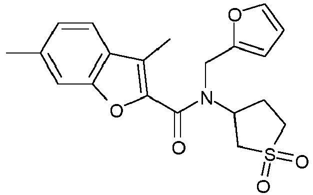 |
| 16 | 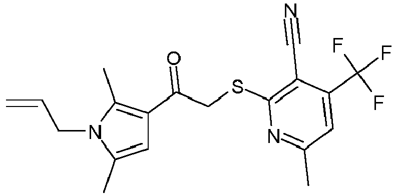 |
| 17 | 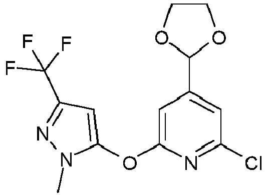 |
| 18 | 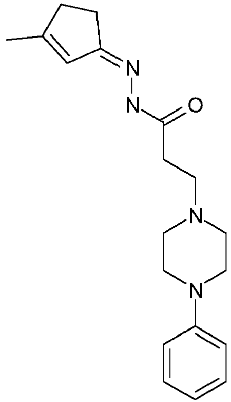 |
| 19 | 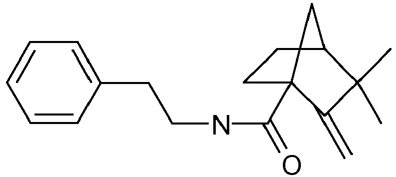 |

6. Verwendung eines Modulators für den TRPM8-Rezeptor, wobei der Modulator gemäß einem der Ausführungsformen 1 bis 4 definiert ist, zur Induktion von Kältegefühl bei Mensch und/oder Tier.
7. Verwendung eines Modulators für den TRPM8-Rezeptor, wobei der Modulator gemäß einem der Ausführungsformen 1 bis 4 definiert ist, als aktiven Bestandteil eines pharmazeutischen Mittels.
8. Verwendung eines Modulators für den TRPM8-Rezeptor, wobei der Modulator gemäß einem der Ausführungsformen 1 bis 4 definiert ist, zur Behandlung von Prostatakarzinomen, zur Behandlung von Blasenschwäche oder in der Schmerztherapie.
9. Verwendung eines Modulators für den TRPM8-Rezeptor, wobei der Modulator gemäß einem der Ausführungsformen 1 bis 4 definiert ist, als Insekten-Repellent oder Insektizid.
10. Verwendung eines Modulators für den TRPM8-Rezeptor, wobei der Modulator gemäß einem der Ausführungsformen 1 bis 4 definiert ist, zur Induktion eines Kältegefühls in einer Verpackung
11. Verwendung eines Modulators für den TRPM8-Rezeptor, wobei der Modulator gemäß einem der Ausführungsformen 1 bis 4 definiert ist, zur Induktion eines Kältegefühls in einem Textil
12. Stoff gemäß der Definition nach einem der Ausführungsformen 1 bis 4 zur Verwendung als Mediator des TRMP8 Rezeptors.
13. Mittel enthaltend wenigstens eine Verbindung nach einem der Ausführungsformen 1 bis 4.
14. Mittel nach Ausführungsform 12, ausgewählt unter
a) pharmazeutischen Mitteln, wie Antitumormittel, Mittel zur Behandlung von Erkrankungen der Blase, Schmerzmittel;
b) Nahrungsmitteln, wie Eis, Mousse, Creme, Getränke, Süßwaren,
c) Mundpflegemitteln, wie Zahnpasta, Mundwasser, Kaugummi,
d) Haut- oder Haarpflegemitteln, wie Sonnencreme, Sonnenbrandcreme, Lotionen, Shampoos, Pflaster,
e) Insektenrepellents, Insektiziden.
15. Textilprodukte, wie z.B. Hemden, Hosen, Socken, Handtücher, ausgerüstet mit wenigstens einer Verbindung nach einem der Ausführungsformen 1 bis 4.
16. Verpackungsmaterial, welches mit wenigstens einer Verbindung nach einem der Ausführungsformen 1 bis 4 assoziiert ist.

## Patentansprüche

1. Verfahren zur in-vitro Modulation des Kälte-Menthol-Rezeptors TRPM8, wobei man den Rezeptor mit wenigstens einer Verbindung in Kontakt bringt, die ausgewählt ist unter den Verbindungen der Formeln 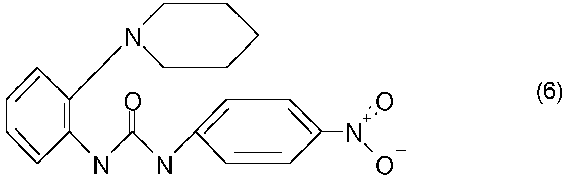 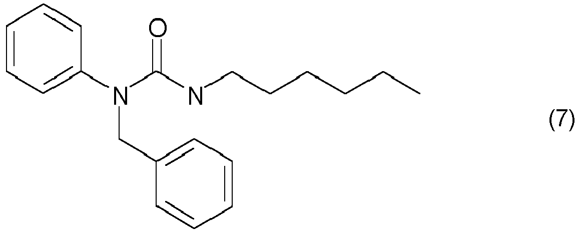 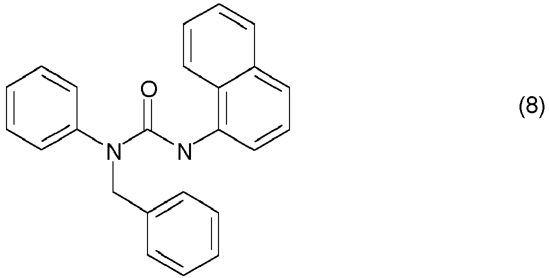 welche in einem zellulären Aktivitätstest unter Verwendung von Zellen, welche den humanen TRPM8-Rezeptor rekombinant exprimieren, die Permeabilität dieser Zellen für Ca²⁺ Ionen modulieren.

2. Verfahren nach Anspruch 1, wobei der Modulator auf die zelluläre Ca²⁺-Ionen-Permeabilität agonistisch wirkt.

3. Verwendung eines Modulators für den TRPM8-Rezeptor, wobei der Modulator gemäß Anspruch 1 definiert ist, zur nicht-therapeutischen Induktion von Kältegefühl bei Mensch und/oder Tier.

4. Verwendung eines Modulators für den TRPM8-Rezeptor der obigen Formel 6, 7 oder 8 als aktiven Bestandteil eines pharmazeutischen Mittels.

5. Modulator für den TRPM8-Rezeptor der obigen Formel 6, 7 oder 8 zur Verwendung bei der Behandlung von Prostatakarzinomen, zur Behandlung von Blasenschwäche oder in der Schmerztherapie.

6. Verwendung eines Modulators für den TRPM8-Rezeptor, wobei der Modulator gemäß Anspruch 1 definiert ist, als Insekten-Repellent oder Insektizid.

7. Verwendung eines Modulators für den TRPM8-Rezeptor, wobei der Modulator gemäß Anspruch 1 definiert ist, zur Induktion eines Kältegefühls in einer Verpackung oder einem Textil.

8. Verbindung der obigen Formel 6, 7 oder 8 zur Verwendung als Medikament.

9. Mittel enthaltend im Gemisch mit üblichen Bestandteilen wenigstens eine Verbindung der obigen Formel 6, 7 oder 8.

10. Mittel nach Anspruch 9, ausgewählt unter
a) pharmazeutischen Mitteln, wie Antitumormittel, Mittel zur Behandlung von Erkrankungen der Blase, Schmerzmittel;
b) Nahrungsmitteln, wie Eis, Mousse, Creme, Getränke, Süßwaren,
c) Mundpflegemitteln, wie Zahnpasta, Mundwasser, Kaugummi,
d) Haut- oder Haarpflegemitteln, wie Sonnencreme, Sonnenbrandcreme, Lotionen, Shampoos, Pflaster,
e) Insektenrepellents, Insektiziden.

11. Textilprodukte, wie Hemden, Hosen, Socken, Handtücher, ausgerüstet mit wenigstens einer Verbindung nach Anspruch 1.

12. Verpackungsmaterial, welches mit wenigstens einer Verbindung der obigen Formel 6, 7 oder 8 assoziiert ist.
